# EUROPEAN PATENT SPECIFICATION

(11) **EP 4 420 599 B1**
(45) Date of publication and mention of the grant of the patent: **11.11.2026**
(21) Application number: 22876989.9
(22) Date of filing: 30.09.2022
(51) Int. Cl.: A61B 5/00, A61F 2/12, A61F 2/48, A61B 5/0205, A61F 2/02

(54) **APPARATUS AND SYSTEM FOR SENSING IMPLANT ABNORMALITY**
VORRICHTUNG UND SYSTEM ZUR ERFASSUNG VON IMPLANTATANOMALIEN
APPAREIL ET SYSTÈME DE DÉTECTION D'ANOMALIE D'IMPLANT

(30) Priority: 03.10.2021 KR 20210131279
(43) Date of publication of application: 28.08.2024
(73) Proprietor: W.Al Co., Ltd., Seoul 06770 (KR)
(72) Inventor: KIM, Jae-Hong, Seoul 06038 (KR)
(74) Representative: Rondano, Davide
(86) International application number: PCT/KR2022/014862
(87) International publication number: WO 2023/055218

(56) References cited:
- EP-A1- 4 295 752
- KR-A- 20200 144 559
- KR-B1- 102 260 203
- KR-B1- 102 324 101
- US-A1- 2009 012 372
- US-A1- 2011 077 736
- US-A1- 2017 181 825
- US-A1- 2018 036 115
- US-A1- 2018 085 210
- US-A1- 2021 038 367

## Description

### BACKGROUND

### Technical Field

The present disclosure relates to a system and an apparatus for sensing an abnormality of an implant.

### Description of Related Technology

A breast with beauty and volume is the shared wishes of most women. The implant is one of the easiest and most effective methods to realize the wishes of such women.

The breast reconstruction employing the implant is a method of securing a space for inserting the implant in a breast and increasing the volume of the breast by inserting the implant in the secured space.

Such breast implants are generally manufactured using a cohesive gel that is a silicone having a high cohesive force. The cohesive gel has an advantage in that an outer cover (shell) is strong and a shape is naturally formed after a surgery, remedying disadvantages of a conventional silicone gel (see, for example, Korean Pat. No. 10-1235284, Korean Pat. No. 10-1966979, and Japanese Pat. Laid-Open No. 2010-534551).

Popular side effects seen after a surgery for reconstructing a breast with an implant include rupture and capsular contracture.

When a rupture is generated and a silicone component is remained in the body for a long time, the silicone component may permeate through the breast tissue, possibly causing a breast feeding hard and a difficulty in a breast cancer examination. Sometimes the silicone penetrates into a lymph node due to the rupture of the implant, and in this case, the penetrated silicone may be incompletely removed and a lymphedema after removing the silicone or a spreading of the silicone to the whole body may occur.

The capsular contracture is a phenomenon generated from thickening of a capsule newly formed surrounding an implant, which is generated within the first two years after a surgery, which is one of the most popular side effects of the breast reconstruction employing the implant. A capsular contracture causes deformation and pain, and in a worse case, a reoperation is needed to remove the implant and to remove the capsule.

In order to examine the two representative side effects after inserting the implant, a patient needs to visit a hospital to consult a doctor and to carry out an image medical inspection such as an ultrasound or an MRI. Even without a particular symptom, it is recommended to carry out an ultrasound examination every year after inserting the implant.

In order to sense abnormalities in such breast implants, for example, US Pat. Pub. No 2009/0012372 describes a system and a method for sensing for the rupture of an implant.

However, when the abnormality of the implant is determined simply by attaching a sensor on the implant and receiving an abnormality signal from the sensor, there may be a risk of a false positive or a false negative.

Particularly, although it is problematic that no abnormality is determined even with an actual abnormality in the case of, for example, a breast implant, it would be worse if an abnormality is determined without an actual abnormality because an operation may be needed to remove a normal breast implant and to insert a new breast implant.

An apparatus for sensing an abnormality of an implant according to the preamble of independent claim 1 is known from US 2017/181825 A1.

### SUMMARY

It is an object of the present disclosure to provide an apparatus for sensing an abnormality of an implant, which can minimize a false positive and a false negative by sensing the abnormality of the implant in an effective manner after a surgery.

It is another object of the present disclosure to provide a system for sensing an abnormality of an implant, which can minimize a false positive and a false negative by sensing the abnormality of the implant in an effective manner after a surgery.

The challenges to be addressed by the present disclosure are not limited to those mentioned above, and other unmentioned problems can be clearly understood by those skilled in the art from the following description.

According to at least one embodiment of the present disclosure, provided is an apparatus for sensing an implant abnormality, which includes: a wireless power transmitting unit for wirelessly transmitting power to at least one sensor module mounted on an implant inserted into the human body; a sensor signal receiving unit for receiving a pressure signal indicating a pressure applied to the implant from the sensor module; a storing unit for storing a predetermined reference pressure value and a first tolerance; an abnormality determining unit for determining whether the implant is abnormal based on the pressure signal received by the sensor signal receiving unit, and the reference pressure value and the first tolerance stored in the storing unit; a determination signal output unit for outputting the determination signal indicating whether the implant is abnormal, which is determined based on the pressure signal, and the reference pressure value and the first tolerance by the abnormality determining unit; a control unit for controlling the wireless power transmitting unit, the sensor signal receiving unit, the storing unit, the abnormality determining unit, and the determination signal output unit; and a power source unit for supplying the power to the wireless power transmitting unit, the sensor signal receiving unit, the storing unit, the abnormality determining unit, and the determination signal output unit through the control unit, in which the control unit receives a first pressure signal from the sensor module according to a predetermined period or according to an arbitrary manipulation of a user through the sensor signal receiving unit, compares a first pressure value indicated by the first pressure signal, and a reference pressure value through the abnormality determining unit, receives a second pressure signal from the sensor module after a predetermined first time lapse through the sensor signal receiving unit when an absolute value of a difference between the first pressure value and the reference pressure value is larger than a first tolerance, and compares a second pressure value indicated by a second pressure signal and a reference pressure value through the abnormality determining unit, and outputs a determination signal indicating whether the implant is abnormal through the determination signal output unit when an absolute value of a difference between the second pressure value and the reference pressure value is larger than the first tolerance.

**In** at least one embodiment of the present disclosure, the storing unit further stores a second tolerance, and the control unit compares an absolute value of a difference between the first pressure value and the second pressure value, and the second tolerance through the abnormality determining unit when the second pressure value is larger than the reference pressure value, and outputs the determination signal indicating whether the implant is abnormal through the determination signal output unit when the ab solute value of the difference between the fist pressure value and the second pressure value is smaller than a second tolerance.

**In** at least one embodiment of the present disclosure, the control unit ignores the predetermined period when the first pressure value is larger than the reference pressure value, and receives a second pressure signal from the sensor module after the first time lapse, when receiving a first pressure signal from the sensor module according to a predetermined period, and ignores the manipulation of the user when the first pressure value is larger than the reference pressure value, and receives a second pressure signal from the sensor module after the first time lapse, when receiving the first pressure signal from the sensor module according to an arbitrary manipulation of a user.

**In** at least one embodiment of the present disclosure, the sensor module includes a plurality of sensor modules, and the storing unit sets and stores the first tolerance differently for each of the plurality of sensor modules.

In at least one embodiment of the present disclosure, the sensor module includes a plurality of sensor modules, and the storing unit sets and stores the first tolerance for any one sensor module among the plurality of sensor modules differently from other sensor modules.

According to at least one embodiment of the present disclosure, provided is an apparatus for sensing an implant abnormality, which includes: wireless power transmitting unit for wirelessly transmitting power to at least one sensor module mounted on an implant inserted into the human body; a sensor signal receiving unit for receiving a pressure signal indicating a pressure applied to the implant from the sensor module; a storing unit for storing a predetermined first tolerance; an abnormality determining unit for determining whether the implant is abnormal based on the pressure signal received by the sensor signal receiving unit, and the first tolerance stored in the storing unit; a determination signal output unit for outputting the determination signal indicating whether the implant is abnormal, which is determined based on the pressure signal, and the first tolerance by the abnormality determining unit; a control unit for controlling the wireless power transmitting unit, the sensor signal receiving unit, the storing unit, the abnormality determination unit and the determination signal output unit; and a power source unit for supplying the power to the wireless power transmitting unit, the sensor signal receiving unit, the storing unit, the abnormality determination unit, and the determination signal output unit through the control unit, in which the control unit receives a first pressure signal from the sensor module according to a predetermined period or according to an arbitrary manipulation of a user through the sensor signal receiving unit, compares a first change amount indicating a difference between a previous pressure signal of the first pressure signal and the first pressure signal, and the first tolerance through the abnormality determining unit, and receives a second pressure signal from the sensor module after a predetermined first time lapse through the sensor signal receiving unit when the first change amount is larger than the first tolerance, compares a second change amount indicating a difference between the first pressure signal and the second pressure signal, and a first tolerance through the abnormality determining unit, and outputs the determination signal indicating whether the implant is abnormal through the determination signal output unit when the second change amount is larger than the first tolerance.

In at least one embodiment of the present disclosure, the storing unit further stores a predetermined second tolerance, and the control unit compares an absolute value of a difference between the first change amount and the second change amount, and the second tolerance through the abnormality determining unit when the second change amount is larger than the first tolerance, and outputs the determination signal indicating whether the implant is abnormal through the determination signal output unit when the absolute value of the difference between the first change amount and the second change amount is smaller than the second tolerance.

In at least one embodiment of the present disclosure, the control unit ignores the predetermined period when the first change amount is larger than the first tolerance, and receives a second pressure signal from the sensor module after the first time lapse, when receiving a first pressure signal from the sensor module according to a predetermined period, and ignores the manipulation of the user when the first change amount is larger than the first tolerance, and receives a second pressure signal from the sensor module after the first time lapse, when receiving the first pressure signal from the sensor module according to an arbitrary manipulation of a user.

In at least one embodiment of the present disclosure, the sensor module includes a plurality of sensor modules, and the storing unit sets and stores the first tolerance differently for each of the plurality of sensor modules.

In at least one embodiment of the present disclosure, the sensor module includes a plurality of sensor modules, and the storing unit sets and stores the first tolerance for any one sensor module among the plurality of sensor modules differently from other sensor modules.

In at least one embodiment of the present disclosure, the sensor signal receiving unit further receives a biometric signal representing at least one of heart rate, pulse, body temperature, blood pressure, respiratory rate, and blood sugar from the sensor module or the plurality of sensor modules, and the storing unit further stores the biometric signal received by the sensor signal receiving unit.

According to at least one embodiment of the present disclosure, provided is a system for sensing an implant abnormality, which includes: a user terminal receiving a pressure signal indicating a pressured applied to an implant from at least one sensor module mounted on the implant inserted into the human body, and transmitting the received pressure signal to the outside; and a remote server receiving the pressure signal from the user terminal, and determining whether the implant is abnormal based on the received pressure signal, in which the user terminal includes a wireless power transmitting unit for wirelessly transmitting power to the sensor module, a sensor signal receiving unit for receiving a pressure signal indicating a pressure applied to the implant from the sensor module, a signal transmitting unit for transmitting the pressure signal received by the sensor signal receiving unit to the remote server, a terminal control unit for controlling the wireless power transmitting unit, the sensor signal receiving unit, and the signal transmitting unit, and a terminal power source unit for supplying power to the wireless power transmitting unit, the sensor signal receiving unit, and the signal transmitting unit through the terminal control unit, the remote server includes a signal receiving unit for receiving a pressure signal from the user terminal, a storing unit for storing a predetermined reference pressure value and a first tolerance, an abnormality determining unit for determining whether the implant is abnormal based on the pressure signal received from the user terminal, and the reference pressure value and the first tolerance stored in the storing unit, a determination signal output unit for outputting a determination signal indicating whether the implant is abnormal determined based on the pressure signal, and the reference pressure value and the first tolerance by the abnormality determining unit, a server control unit for controlling the signal receiving unit, the storing unit, the abnormality determination unit, and the determination signal output unit, and a server power source unit for supplying power to the signal receiving unit, the storing unit, the abnormality determination unit, and the determination signal output unit through the server control unit, and the server control unit receives a first pressure signal from the sensor module according to a predetermined period or according to an arbitrary manipulation of a user through the terminal control unit, compares a first pressure value indicated by the first pressure signal, and a reference pressure value through the abnormality determining unit, receives a second pressure signal from the sensor module after a predetermined first time lapse through the terminal control unit when an abnormal value of a difference between the first pressure value and the reference pressure value is larger than a first tolerance, compares a second pressure value indicated by a second pressure signal and a reference pressure value through the abnormality determining unit, and outputs a determination signal indicating whether the implant is abnormal through the determination signal output unit when an absolute value of a difference between the second pressure value and the reference pressure value is larger than the first tolerance.

According to at least one embodiment of the present disclosure, provided is a system for sensing an implant abnormality, which includes: a user terminal receiving a pressure signal indicating a pressured applied to an implant from at least one sensor module mounted on the implant inserted into the human body, and transmitting the received pressure signal to the outside; and a remote server receiving the pressure signal from the user terminal, and determining whether the implant is abnormal based on the received pressure signal, in which the user terminal includes a wireless power transmitting unit for wirelessly transmitting power to the sensor module, a sensor signal receiving unit for receiving a pressure signal indicating a pressure applied to the implant from the sensor module, a signal transmitting unit for transmitting the pressure signal received by the sensor signal receiving unit to the remote server, a terminal control unit for controlling the wireless power transmitting unit, the sensor signal receiving unit, and the signal transmitting unit, and a terminal power source unit for supplying power to the wireless power transmitting unit, the sensor signal receiving unit, and the signal transmitting unit through the terminal control unit, the remote server includes a signal receiving unit for receiving a pressure signal from the user terminal, a storing unit for storing a predetermined first tolerance, an abnormality determining unit for determining whether the implant is abnormal based on the pressure signal received from the user terminal, and the first tolerance stored in the storing unit, a determination signal output unit for outputting the determination signal indicating whether the implant is abnormal, which is determined based on the pressure signal, and the first tolerance by the abnormality determining unit, a server control unit for controlling the signal receiving unit, the storing unit, the abnormality determination unit, and the determination signal output unit, and a server power source unit for supplying power to the signal receiving unit, the storing unit, the abnormality determination unit, and the determination signal output unit through the server control unit, and the server control unit receives a first pressure signal from the sensor module according to a predetermined period or according to an arbitrary manipulation of a user through the terminal control unit, compares a first change amount indicating a difference between a previous pressure signal of the first pressure signal and the first pressure signal, and the first tolerance through the abnormality determining unit, and receives a second pressure signal from the sensor module after a predetermined first time lapse through the terminal control unit when the first change amount is larger than the first tolerance, compares a second change amount indicating a difference between the first pressure signal and the second pressure signal, and a first tolerance through the abnormality determining unit, and outputs the determination signal indicating whether the implant is abnormal through the determination signal output unit when the second change amount is larger than the first tolerance.

In at least one embodiment of the present disclosure, the sensor signal receiving unit further receives a biometric signal representing at least one of heart rate, pulse, body temperature, blood pressure, respiratory rate, and blood sugar from the sensor module, the signal transmitting unit transmits the biometric signal received by the sensor signal receiving unit to the remote server, the signal receiving unit receives the biometric signal from the user terminal, and the storing unit further stores the biometric signal received from the user terminal.

According to at least one embodiment of the present disclosure, provided is an apparatus for sensing an implant abnormality, which includes: a wireless power transmitting unit for wirelessly transmitting power to a plurality of sensor modules mounted on an implant inserted into the human body; a sensor signal receiving unit for receiving a pressure signal indicating a pressure applied to the implant from each of the plurality of sensor modules; a storing unit for storing a reference pressure value predetermined for each of the plurality of sensor modules; an abnormality determining unit for determining whether the implant is abnormal based on the pressure signal received for each of the plurality of sensor modules and the reference pressure value stored in the storing unit for each of the plurality of sensor modules; a determination signal output unit for outputting the determination signal indicating whether the implant is abnormal, which is determined based on the pressure signal and the reference pressure value by the abnormality determining unit; a control unit for controlling the wireless power transmitting unit, the sensor signal receiving unit, the storing unit, the abnormality determination unit and the determination signal output unit; and a power source unit for supplying the power to the wireless power transmitting unit, the sensor signal receiving unit, the storing unit, the abnormality determination unit, and the determination signal output unit through the control unit, in which the storing unit sets and stores the reference pressure value differently for each of the plurality of sensor modules.

In this specification, each embodiment is described independently, but each embodiment can be combined with each other, and the combined embodiments are also included in the scope of the present disclosure.

The foregoing summary is for illustrative purposes only and is not intended to be limiting in any way. In addition to the illustrative aspects, embodiments and features described above, additional aspects, embodiments and features will become apparent by reference to the drawings and the detailed description below.

According to at least one embodiment of the present disclosure, there is an effect in that an apparatus for sensing implant abnormality to minimize the false positive rate and the false negative rate by effectively sensing the abnormality of the implant after the procedure can be provided.

According to at least one embodiment of the present disclosure, there is an effect in that a system for sensing implant abnormality to minimize the false positive rate and the false negative rate by effectively sensing the abnormality of the implant after the procedure can be provided.

The effects of the present disclosure are not limited to those mentioned above, and other effects not mentioned will be clearly understood by those skilled in the art from the description below.

### BRIEF DESCRIPTION OF THE DRAWINGS

Fig. 1 is a schematic diagram of a system and an apparatus for sensing an abnormality of an implant according to at least one embodiment of the present disclosure;
Fig. 2 is a functional block diagram of an apparatus for sensing an abnormality of an implant according to at least one embodiment of the present disclosure;
Figs. 3 and 4 are flowcharts for explaining a method of sensing an abnormality of an implant according to at least one embodiment of the present disclosure;
Fig. 5 is a functional block diagram an apparatus for sensing an abnormality of an implant in a system for sensing an abnormality of an implant according to at least one embodiment of the present disclosure;
Fig. 6 is a functional block diagram a server for sensing an abnormality of an implant in a system for sensing an abnormality of an implant according to at least one embodiment of the present disclosure;
Fig. 7 is a schematic diagram of a breast implant including a sensor patch according to at least one embodiment of the present disclosure;
Figs. 8A and 8B are schematic diagrams patches each including a pressure sensor according to at least one embodiment of the present disclosure;
Fig. 9 is an exploded view of a sensor module according to at least one embodiment of the present disclosure;
Fig. 10A is a schematic diagram for explaining pressures applied to an implant inserted in a breast in at least one embodiment of the present disclosure; and
Fig. 10B is a schematic diagram showing an example of attaching a patch including a sensor module according to at least one embodiment of the present.

### DETAILED DESCRIPTION

Exemplary embodiments of the present disclosure are described in detail below with reference to the accompanying drawings.

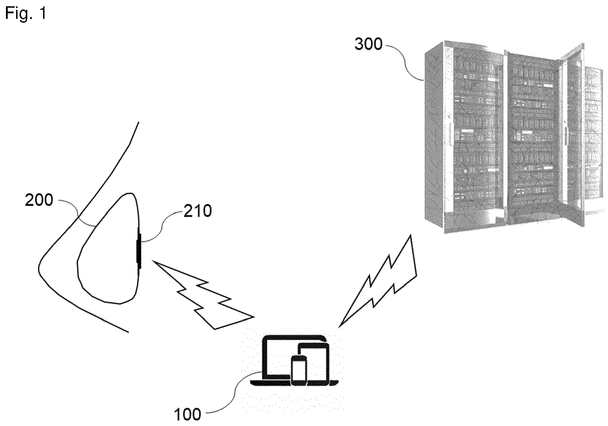

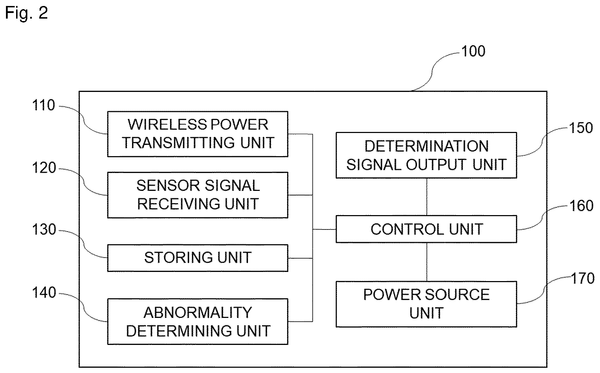

Fig. 1 is a schematic diagram of a system and an apparatus 100 for sensing an abnormality of an implant according to at least one embodiment of the present disclosure. Fig. 2 is a functional block diagram of the apparatus 100 for sensing an abnormality of an implant according to at least one embodiment of the present disclosure.

The system for sensing an abnormality of an implant according to at least one embodiment of the present disclosure includes an apparatus 100 for sensing an abnormality of an implant 200 configured to receive a pressure signal indicating a pressure applied to the implant 200 from at least one sensor module 210 disposed on the implant 200 inserted in a body (breast) and to sense an abnormality of the implant 200 based on the pressure signal received from the sensor module 210 and a server 300 that is remotely connected with the apparatus 100 and configured to receive various pieces of data from the apparatus 100 and to store received data.

In at least one embodiment of the present disclosure, the apparatus 100 is configured to receive a pressure signal indicating a pressure applied to the implant 200 from the sensor module 210 disposed on the implant 200, to output a determination signal by determining an abnormality in the implant 200 based on the pressure signal received from the sensor module 210, and to transmit various pieces of data including the pressure signal and the determination signal to the server 300 to allow the data to be stored in the server 300.

As shown in Fig. 2, the apparatus 100 according to at least one embodiment of the present disclosure includes a wireless power transmitting unit 110 configured to wirelessly transmit a power to the sensor module 210 disposed on the implant 200 that is to be inserted in a body (breast), a sensor signal receiving unit 120 configured to receive the pressure signal indicating the pressure applied to the implant 200 from the sensor module 210, a storing unit 130 configured to store a predetermined reference pressure value and a predetermined first tolerance, an abnormality determining unit 140 configured to determine an abnormality of the implant 200 based on the pressure signal received by the sensor signal receiving unit 120 and the reference pressure value and the first tolerance stored in the storing unit 130, a determination signal output unit 150 configured to output a determination signal indicating an abnormality or no abnormality in the implant 200 determined by the abnormality determining unit 140 based on the pressure signal, the reference pressure value, and the first tolerance, a control unit 160 configured to control the units 110 to 150, and a power source unit 170 configured to supply a power to the units 110 to 150 through the control unit 160.

In at least one embodiment of the present disclosure, the control unit 160 controls the sensor signal receiving unit 120 to receive a first pressure signal from the sensor module 210 in a predetermined (set) period or in an arbitrary manner by an operation of a user, the abnormality determining unit 140 to compare a first pressure value indicated by the first pressure signal and the reference pressure value, when an absolute value of a difference between the first pressure value and the reference pressure value is larger than the first tolerance, the sensor signal receiving unit 120 to receive a second pressure signal from the sensor module 210 after a predetermined first time lapse, the abnormality determining unit 140 to compare a second pressure value indicated by the second pressure signal with the reference pressure value, and when an absolute value of a difference between the second pressure value and the reference pressure value is larger than the first tolerance, the determination signal output unit 150 to output a determination signal indicating an abnormality in the implant 200.

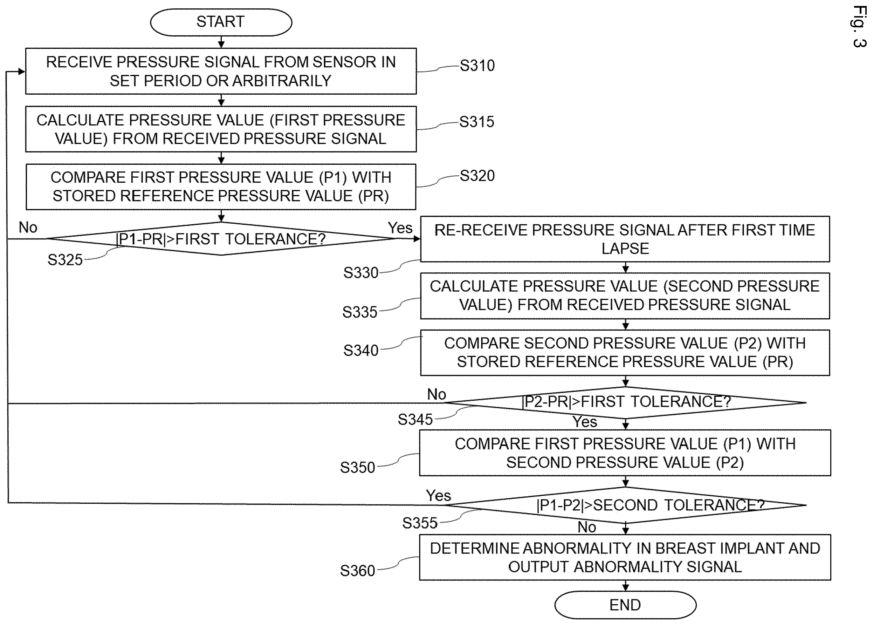

Fig. 3 is a flowchart for explaining a method of sensing an abnormality of the implant 200 according to at least one embodiment of the present disclosure.

In the following descriptions, each operation is performed by the control unit 160 to control each corresponding function module implemented as a program module in a processor and each corresponding hardware component.

In step S310, a pressure signal is received from a sensor (sensor module) of an implant in a predetermined period or by an arbitrary operation of a user.

Thereafter, a pressure value (first pressure value) applied to the implant is calculated from the pressure signal received in step S310 (step S315). The first pressure value calculated in the above manner is then compared with a reference pressure value stored in advance (step S320).

In at least one embodiment of the present disclosure, the reference pressure value is an actual pressure value measured in a predetermined stabilization time after a surgery and stored in a storing unit (memory). The reference pressure value is updated by re-measuring the actual pressure value in a predetermined time period.

That is, the reference pressure value indicates a pressure applied to the implant in a normal state; and therefore, when a pressure value indicated by a received pressure signal is larger or smaller than the reference pressure value by a predetermined amount, it is possible to determine that a rupture or a capsular contracture has occurred in the implant.

As a result of comparing the calculated first pressure value with the stored reference pressure value, if an absolute value of a difference between the first pressure value and the reference pressure value is larger than the stored first tolerance (step S325, Yes), a second pressure signal is received from the sensor module after a predetermined first time lapse (step S330).

A pressure value (second pressure value) is calculated from the received second pressure signal (step S335). The calculated second pressure value is then compared with the stored reference pressure value (step S340).

As a result of comparing the second pressure value with the reference pressure value, if an absolute value of a difference between the second pressure value and the reference pressure value is larger than the first tolerance (step S345, Yes), a determination signal indicating an abnormality in the implant is outputted (step S360).

As large and small pressures can be constantly applied to an implant inserted in a body, if an abnormality is determined with a single measurement, there always exists a risk of a false positive or a false negative.

For example, as a result of comparing the first pressure value with the reference pressure value in step S325, if the absolute value of the difference between the first pressure value and the reference pressure value is larger than the first tolerance, that is, if it is determined that an abnormal pressure is applied to the sensor disposed on the implant, it may be necessary to determine whether such an abnormal pressure is temporary or persisting.

To this end, in at least one embodiment of the present disclosure, once it is determined that the absolute value of the difference between the first pressure value and the reference pressure value is larger than the first tolerance, the second pressure signal is received from the sensor module after the predetermined first time lapse.

In at least one embodiment of the present disclosure, upon receiving the pressure signal from the sensor module in the predetermined period, when the first pressure value is larger than the reference pressure value, the control unit receives the second pressure signal from the sensor module after the first time lapse, ignoring the predetermined period.

In at least one embodiment of the present disclosure, upon receiving the pressure signal from the sensor module by an arbitrary operation of a user, when the first pressure value is larger than the reference pressure value, the control unit receives the second pressure signal from the sensor module after the first time lapse, ignoring the operation of the user.

The first time is a time for determining whether the abnormal pressure is applied to the sensor disposed on the implant temporarily or constantly, which can be set longer or shorter than the predetermined period.

In at least one embodiment of the present disclosure, if the predetermined time is set to t1, the time obtained by dividing t1 by a predetermined integer can be set as the first time.

For example, if t1 is set to 12 hours, 2 hours which is the value of t1 divided by 6, can be set as the first time (sixth rule), and 2.4 hours which is the value of t1 divided by 5, can be set as the first time (quintile method), 3 hours which is the value of t1 divided by 4, can be set as the first time (quarter method), and 4 hours which is the value of t1 divided by 3, can be set as the first time (trichotomous method), and 6 hours which is the value of t1 divided by 2 can be set as the first time (dichotomy).

In at least one embodiment of the present disclosure, the predetermined integer may be set to a value of 6 or more depending on the setting value of t1, and the larger this value is, the shorter the interval to the first time can be set, and the smaller this value, the longer the interval to the first time can be set.

In order to further reduce the risk of the false positive and the false negative, in at least one embodiment of the present disclosure, as a result of comparing the second pressure value with the reference pressure value, if the absolute value of the difference between the second pressure value and the reference pressure value is larger than the first tolerance (step S345, Yes), the first pressure value is compared with the second pressure value (step S350).

As a result of comparing the first pressure value with the second pressure value, if an absolute value of a difference between the first pressure value and the second pressure value is smaller than a second tolerance (step S355, No), a determination signal indicating an abnormality in the implant is outputted (step S360).

The first pressure value is compared with the second pressure value in the above manner because, if there is a big difference between the pressure values before and after the first time, it is possible to determined that the abnormal pressure is more likely applied to the implant in a temporal manner, and if there is a small difference between the pressure values before and after the first time within the second tolerance, it is possible to determine that the abnormal pressure is applied to the implant constantly for the first time, meaning that an abnormality has occurred in the implant.

In this manner, the apparatus 100 according to at least one embodiment of the present disclosure can sense an abnormality in an implant after a surgery in an effective manner, thus minimizing the risk of the false positive and the false negative.

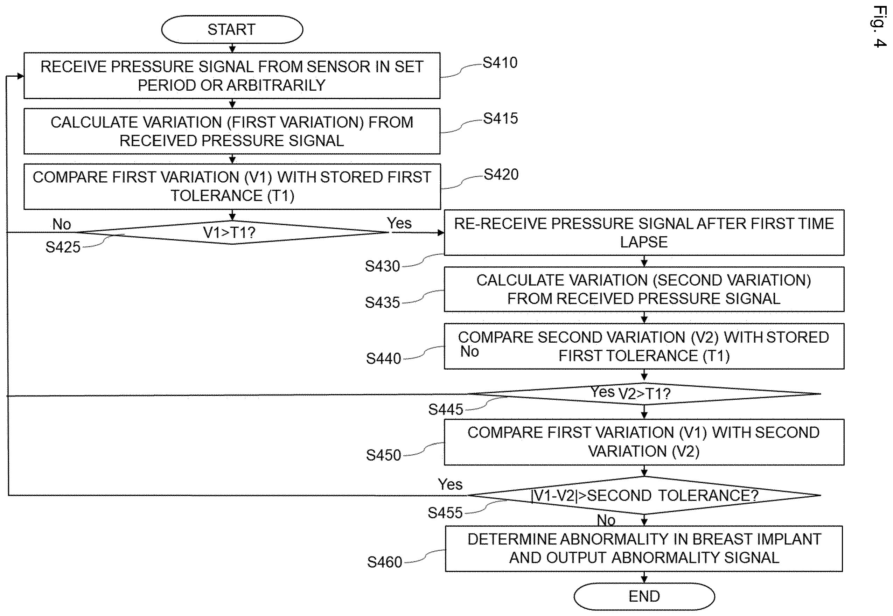

Fig. 4 is a flowchart for explaining a method of sensing an abnormality of the implant 200 according to at least one embodiment of the present disclosure.

In step S410, a pressure signal (first pressure signal) is received from a sensor (sensor module) of an implant in a predetermined period or by an arbitrary operation of a user.

After receiving a series of first pressure signals including at least two first pressure signals, a first variation indicating a difference between a current first pressure signal and a previous first pressure signal is calculated (step S415). The calculated first variance is then compared with a first tolerance stored in advance (step S420).

As a result of comparing the first variance with the first tolerance, if the first variance is larger than the first tolerance (step S425, Yes), a second pressure signal is received from the sensor module after a predetermined first time lapse (step S430).

A second variance indicating a difference between the first pressure signal and the second pressure signal is calculated (step S435). The calculated second variance is then compared with the first tolerance (step S440).

As a result of comparing the second variance with the first tolerance, if the second variance is larger than the first tolerance (step S445, Yes), a determination signal indicating an abnormality in the implant is outputted (step S460).

As large and small pressures can be constantly applied to an implant inserted in a body, if an abnormality is determined with a single measurement, there always exists a risk of a false positive or a false negative.

For example, as a result of comparing the first variance calculated in step S425 with the first tolerance, if the first variance is larger than the first tolerance, that is, if it is determined that an abnormal pressure is applied to the sensor disposed on the implant, it may be necessary to determine whether such an abnormal pressure is temporary or persisting.

To this end, in at least one embodiment of the present disclosure, once it is determined that the first variance is larger than the first the second pressure signal is received from the sensor module after the predetermined first time lapse.

In at least one embodiment of the present disclosure, upon receiving the pressure signal from the sensor module in the predetermined period, when the first variance is larger than the first tolerance, the control unit receives the second pressure signal from the sensor module after the first time lapse, ignoring the predetermined period.

In at least one embodiment of the present disclosure, upon receiving the pressure signal from the sensor module by an arbitrary operation of a user, when the first variance is larger than the first tolerance, the control unit receives the second pressure signal from the sensor module after the first time lapse, ignoring the operation of the user.

The first time is a time for determining whether the abnormal pressure is applied to the sensor disposed on the implant temporarily or constantly, which can be set equal to or longer or shorter than the predetermined period.

In order to further reduce the risk of the false positive and the false negative, in at least one embodiment of the present disclosure, as a result of comparing the second variance with the first tolerance, if the second variance is larger than the first tolerance (step S445, Yes), the first variance is compared with the second variance (step S450).

As a result of comparing the first variance with the second variance, if an absolute value of a difference between the first variance and the second variance is smaller than a second tolerance (step S455, No), a determination signal indicating an abnormality in the implant is outputted (step S460).

The first variance is compared with the second variance in the above manner because, if there is a big difference between the pressure values before and after the first time, it is possible to determined that the abnormal pressure is more likely applied to the implant in a temporal manner, and if there is a small difference between the pressure values before and after the first time within the second tolerance, it is possible to determine that the abnormal pressure is applied to the implant constantly for the first time, meaning that an abnormality has occurred in the implant.

In this manner, the apparatus 100 according to at least one embodiment of the present disclosure can sense an abnormality in an implant after a surgery in an effective manner, thus minimizing the risk of the false positive and the false negative.

In at least one embodiment of the present disclosure, the sensor signal receiving unit 120 further receives a bio-signal indicating at least one of heart rate, pulse, body temperature, blood pressure, breathing rate, and blood glucose from the sensor module 210, and the storing unit 130 further stores the bio-signal received by the sensor signal receiving unit 120.

In at least one embodiment of the present disclosure, the control unit 160 stores various pieces of data including the pressure signal, the determination signal, and the bio-signal in the storing unit 130, and in at least one embodiment of the present disclosure, the control unit 160 transmits the data to the server 300 so that the server 300 stores therein the data.

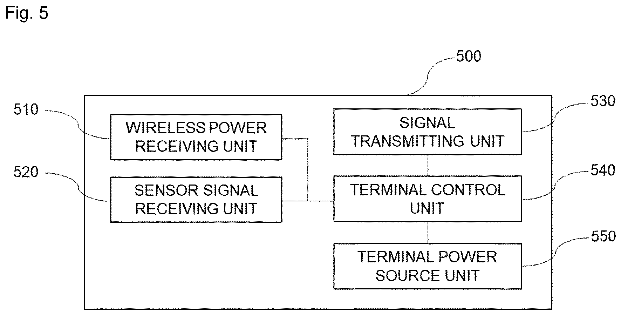

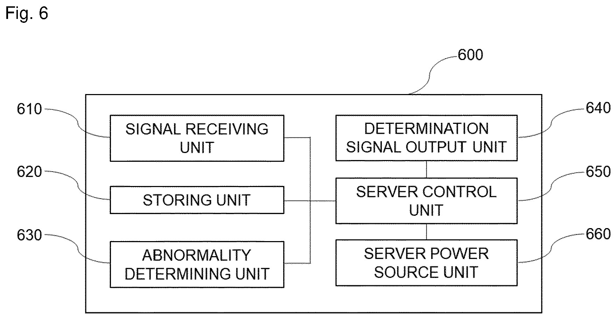

Fig. 5 is a functional block diagram an apparatus for sensing an abnormality of an implant in a system for sensing an abnormality of an implant according to at least one embodiment of the present disclosure. Fig. 6 is a functional block diagram a server for sensing an abnormality of an implant in a system for sensing an abnormality of an implant according to at least one embodiment of the present disclosure.

The system for sensing an abnormality of an implant according to at least one embodiment of the present disclosure includes an apparatus (user terminal) 500 for sensing an abnormality in an implant configured to receive a pressure signal indicating a pressure applied to an implant from at least one sensor module dispose on the implant inserted in a body and to transmit the pressure signal to outside and a server (remote server) 600 for sensing an abnormality of an implant configured to receive the pressure signal from the user terminal 500 and to determine an abnormality in the implant based on the received pressure signal.

In at least one embodiment of the present disclosure, the user terminal 500 includes a wireless power transmitting unit 510 configured to wirelessly transmit a power to the sensor module 210, a sensor signal receiving unit 520 configured to receive a pressure signal indicating a pressure applied to the implant 200 from the sensor module 210, a signal transmitting unit 530 configured to transmit the pressure signal received by the sensor signal receiving unit 520 to the remote server 600, a terminal control unit 540 configured to control the units 510 to 530, and a terminal power source unit 550 configured to supply a power to the units 510 to 530 through the terminal control unit 540.

In at least one embodiment of the present disclosure, the remote server 600 includes a signal receiving unit 610 from the user terminal 500, a storing unit 620 configured to store a predetermined reference pressure value and a predetermined first tolerance, an abnormality determining unit 630 configured to determine an abnormality of the implant based on the pressure signal received from the user terminal 500 and the reference pressure value and the first tolerance stored in the storing unit 620, a determination signal output unit 640 configured to output a determination signal indicating an abnormality or no abnormality in the implant determined by the abnormality determining unit 630 based on the pressure signal, the reference pressure value, and the first tolerance, a server control unit 650 configured to control the units 610 to 640, and a server power source unit 660 configured to supply a power to the units 610 to 640 through the server control unit 650.

In at least one embodiment of the present disclosure, the server control unit 650 receives a first pressure signal through the terminal control unit 540, the first pressure signal is received from the sensor module 210 in a predetermined (set) period or in an arbitrary manner by an operation of a user, the abnormality determining unit 630 to compare a first pressure value indicated by the first pressure signal and the reference pressure value, when an absolute value of a difference between the first pressure value and the reference pressure value is larger than the first tolerance, receive a second pressure signal through the terminal control unit 540, the second pressure signal is received from the sensor module 210 after a predetermined first time lapse, the abnormality determining unit 630 to compare a second pressure value indicated by the second pressure signal with the reference pressure value, and when an absolute value of a difference between the second pressure value and the reference pressure value is larger than the first tolerance, the determination signal output unit 640 to output a determination signal indicating an abnormality in the implant 200.

In at least one embodiment of the present disclosure, the remote server 600 includes a receiving unit 610, a storing unit 620, an abnormality determining unit 630, a determination signal output unit 640, a server control unit 650, and a server power source unit 660. The signal receiving unit 610 is configured to receive the pressure signal from the user terminal 500, the storing unit 620 is configured to store a predetermined first tolerance, the abnormality determining unit 630 is configured to determine an abnormality in the implant 200 based on the pressure signal received from the user terminal 500 and the first tolerance stored in the storing unit 620, the determination signal output unit 640 is configured to output a determination signal indicating an abnormality in the implant 200 determined by the abnormality determining unit 630 based on the pressure signal and the first tolerance, the server control unit 650 is configured to control the units 610 to 640, and the server power source unit 660 is configured to supply a power to the units 610 to 640 through the server control unit 650.

In at least one embodiment of the present disclosure, the server control unit 650 receives a first pressure signal through the terminal control unit 540, the first pressure signal is received from the sensor module 210 in a predetermined (set) period or in an arbitrary manner by an operation of a user, the abnormality determining unit 630 to compare a first variance indicating a difference between the current first pressure signal and the previous first pressure signal with the first tolerance, when the first variance is larger than the first tolerance, receive a second pressure signal through the terminal control unit 540, the second pressure signal is received from the sensor module 210 after a predetermined first time lapse, the abnormality determining unit 630 to compare a second variance indicating a difference between the first pressure signal and the second pressure signal, and if the second variance is larger than the first tolerance, the determination signal output unit 640 to output a determination signal indicating an abnormality in the implant 200.

In at least one embodiment of the present disclosure, the sensor signal receiving unit 520 further receives a bio-signal indicating at least one of heart rate, pulse, body temperature, blood pressure, breathing rate, and blood glucose from the sensor module 210, and the signal transmitting unit 530 transmits the bio-signal received by the sensor signal receiving unit 520 alone or with the pressure signal and the determination signal to outside.

In this manner, the user terminal 500 shown in Fig. 5 is configured to receive the pressure signal from the sensor module 210 and to transmit the pressure signal to the remote server 600 without storing the pressure signal or determining an abnormality of the implant, which makes the structure simple.

The pressure signal transmitted from the user terminal 500 is stored in the remote server 600 so that the abnormality of the implant is determined in the remote server 600. When the bio-signal is transmitted from the user terminal 500, the remote server 600 receives and stores the bio-signal.

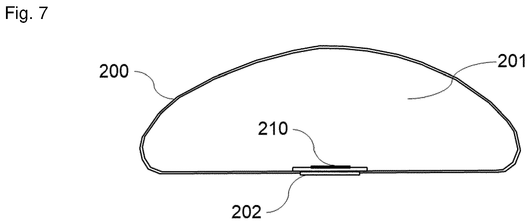

Fig. 7 is a schematic diagram of the implant 200 including a sensor patch according to at least one embodiment of the present disclosure.

The implant 200 according to at least one embodiment of the present disclosure is manufactured with, for example, a silicone material and is used for a plastic surgery of a breast, a hip, or the like, which is inserted in a body and forms and maintains a consistent shape in the body.

Although a breast implant for reconstructing a breast is explained as an example in the present specification, the implant and the apparatus for sensing an abnormality of an implant according to at least one embodiment of the present disclosure can be applied to most implants including a shell and a gel-type filler and being inserted in a body for a specific purpose.

As shown in Fig. 7, the implant 200 according to at least one embodiment of the present disclosure includes a shell 201 forming an outer cover of teardrop type or round type, a silicone gel as a filler injected into the shell 201, and a patch 202 for sealing an injection hole for injecting the silicone gel.

The patch 202 according to at least one embodiment of the present disclosure includes a sensor module 210 including a pressure sensor for sensing a pressure applied to the implant 200.

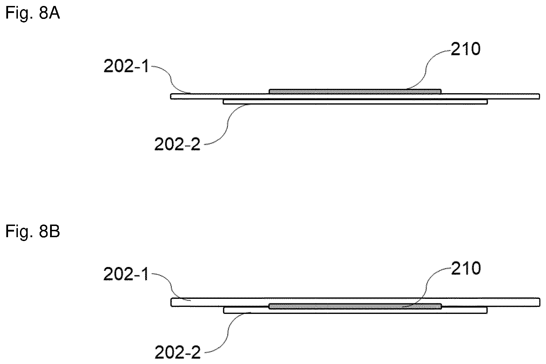

Figs. 8A and 8B are schematic diagrams patches each including a pressure sensor according to at least one embodiment of the present disclosure.

The patch 202 includes a first barrier (inner barrier) 202-1 forming a first side facing inside the implant 200 upon being attached on the implant 200 with the filler of the gel state injected, a second barrier (outer barrier) 202-2 facing the first barrier 202-1 and forming a second side opposite to the first side. The sensor module 210 is disposed on the first side of the first barrier 202-1.

In the patch 202 shown in Fig. 8B, the sensor module 210 is disposed between the first barrier 202-1 and the second barrier 202-2.

Although the first barrier 202-1 and the second barrier 202-2 are shown separately in Figs. 8A and 8B for the sake of explanation, the patch 202 is a unit member formed by a first barrier 211 and a second barrier 212 making contact with each other. The first barrier 202-1 forms the inner side of the patch 202, and the second barrier 202-2 forms the outer side of the patch 202.

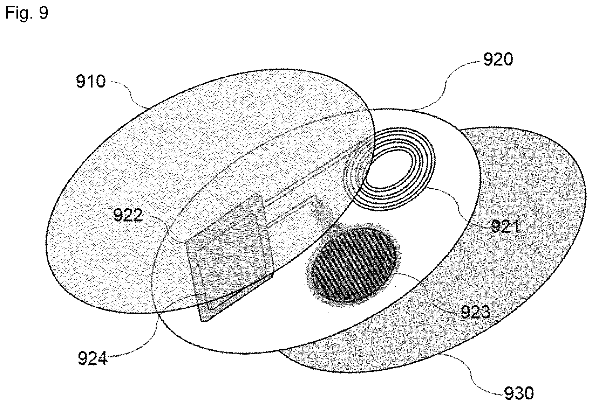

Fig. 9 is an exploded view of the sensor module 210 according to at least one embodiment of the present disclosure.

As shown in Fig. 9, the sensor module 210 is formed as a film including two protective films 910, 930 and a substrate 920 between the two protective films 910, 930. On the substrate 920, at least one pressure sensor 923 for sensing a pressure applied on the implant and outputting a pressure signal, a controller 922 for transmitting the pressure signal from the pressure sensor 923 to the outside, and a wireless power receiving unit 921 for receiving a power from the outside and providing the power to the controller 922.

The pressure sensor 923 is an element that, when a predetermined pressure or force is applied, generates a signal proportional to the pressure or the force applied. The pressure sensor 923 includes a diaphragm sensor that curves according to the pressure or the force applied, a force sensing resistor (FSR) including two conductive plate and a conductive polymer between the two conductive plate, and a piezoelectric element that generates a voltage when a pressure is applied.

A tension sensor that senses a tension of the shell of the implant can be used instead of the pressure sensor; however, as a change of the tension is also a physical amount proportional to a change of the pressure due to the rupture or the capsular contracture of the implant, the tension sensor is considered as a type of the pressure sensor in the present specification.

In at least one embodiment of the present disclosure, the sensor module 210 further includes a battery 924 for storing the power received by the wireless power receiving unit 921 in a wireless manner.

That is, the sensor module 210 according to at least one embodiment of the present disclosure can operate either by receiving the power in a wireless manner without a battery or by charging a battery with the power received in a wireless manner.

In at least one embodiment of the present disclosure, the sensor module 210 includes at least one of a heart rate sensor, a pulse sensor, a body temperature sensor, a blood pressure sensor, a breathing rate sensor, and a blood glucose sensor.

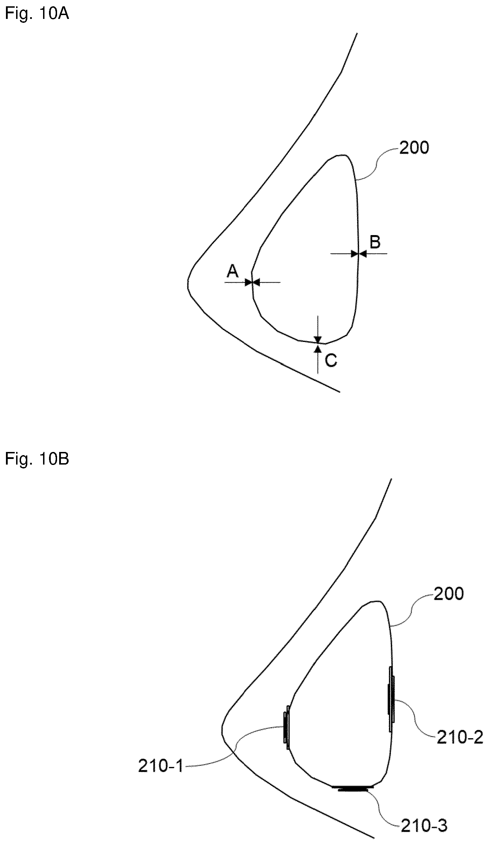

Fig. 10A is a schematic diagram for explaining pressures applied to the implant 200 inserted in a breast in at least one embodiment of the present disclosure. Fig. 10B is a schematic diagram showing an example of attaching a patch including a sensor module according to at least one embodiment of the present.

As shown in Fig. 10A, when the implant 200 is inserted in a breast, in a normal state in which no external pressure is applied on the breast, a pressure A is applied on the implant 200 from outside a body, a pressure B is applied on the implant 200 from or against inside the body, and pressure C is applied on the implant 200 due to the gravity.

Typically, a silicone gel is inserted in a shell of an implant. The implant is sealed by a patch at a position on the bottom of the implant (near a position indicated by B) in a manner that a consistent tension is maintained. However, when a part of the implant is ruptured or a capsular contracture is generated at a part of the implant, at least one of the pressures A, B, and C is changed.

In the case of a cohesive-gel implant, which is widely used in these days, even if a part of the implant is ruptured after inserting the implant in the breast, a patient can hardly feel an abnormality, which is referred to as a silent rupture. That is, the cohesive gel keeps the gel state even when the implant is ruptured.

An image inspection by a magnetic resonance imaging (MRI) is so far one of the most precise methods to inspect a rupture of the implant. However, the MRI itself still shows a false positive and a false negative and can be a burden to a patient in terms of the cost.

When a foreign material comes into a body, a capsule is formed around the foreign material. Such capsule excessively formed and hardened causes a capsular contracture.

When an implant is inserted in a breast, the blood platelet in the blood is activated and a material called the transforming growth factor β (TGF-β) is secreted. The TGF-β takes a role of gathering monocytes around the implant where an inflammation is generated. The monocyte is a sort of the white blood cell, which develops into a macrophage near the inflammation and secretes the TGF-β again. This results in a fibroblast near the inflammation and a collagen synthesized from the fibroblast generates the capsular contracture.

As shown in Fig. 10A, when an implant is inserted in a breast, in a normal state in which no external pressure is applied on the breast, a pressure A is applied on the implant 200 from outside a body, a pressure B is applied on the implant 200 from or against inside the body, and pressure C is applied on the implant 200 due to the gravity. Therefore, with a patch including a pressure sensor disposed on at least one of sites A, B, and C, it is possible to notify a user of an abnormality of the implant by sending a change of the pressure when the abnormality occurred on the implant.

As shown in Fig. 10B, a patch including a sensor module 210-1 can be disposed on the site A, a patch including a sensor module 210-2 can be disposed on the site B, and a patch including a sensor module 210-3 can be disposed on the site C.

In at least one embodiment of the present disclosure, the apparatus for sensing the abnormality of the implant and the system for sensing the abnormality of the implant store the reference pressure and the first tolerance for each of a plurality of positions, for example, the sites A, B, and C shown in Fig. 10A, in the memory.

The apparatus for sensing the abnormality of the implant and the system for sensing the abnormality of the implant according to at least one embodiment of the present disclosure are supposed to output an abnormality of the implant when, for example, a difference between at least one of a plurality of pressure signals received respectively from the sensor modules disposed at a plurality of sites and a reference pressure stored in association with the corresponding site exceeds a predetermined value.

The apparatus for sensing the abnormality of the implant and the system for sensing the abnormality of the implant according to at least one embodiment of the present disclosure can set the first tolerance for determining the abnormality in a pressure signal received from one sensor module larger than the first tolerances for determining the abnormality in pressure signals received from the other sensor modules.

With this configuration, by outputting the abnormality upon sensing an abnormality in one site when the magnitude of the abnormality is relatively large and not outputting the abnormality when the magnitude of the abnormality is relatively small and when the abnormality is in an acceptable range, it is possible to prevent from unnecessarily outputting the abnormality.

The apparatus for sensing the abnormality of the implant and the system for sensing the abnormality of the implant according to at least one embodiment of the present disclosure can set different first tolerances for a plurality of sites from each other to determine the abnormality.

The apparatus for sensing the abnormality of the implant and the system for sensing the abnormality of the implant according to at least one embodiment of the present disclosure can set, for example, the first tolerance for the bottom of the implant (the site C in Fig. 10A) larger or smaller than those for the other sites.

With this configuration, it is possible to make a proper determination considering the change in the pressure depending on the site of the implant.

According to at least one embodiment of the present disclosure, when there is a change in the pressure due to the rupture or the capsular contracture, a user is notified of the rupture or the capsular contracture of the implant according to the change of the pressure, to enable the user to take a proper action at the right time. This can help the user to remove an anxiety about maintaining the breast with safety for a long time after inserting the implant in the breast, and at the same time, to relieve an inconvenience of taking an ultrasound inspection or an **MRI** inspection on a regular basis.

As described above, according to at least one embodiment of the present disclosure, it is possible to provide an apparatus for sensing an abnormality of an implant, which can minimize a false positive and a false negative by sensing the abnormality of the implant in an effective manner after a surgery.

Further, according to at least one embodiment of the present disclosure, it is possible to provide a system for sensing an abnormality of an implant, which can minimize a false positive and a false negative by sensing the abnormality of the implant in an effective manner after a surgery.

In other words, through the present disclosure, patients can quickly sense an abnormal state of the implant in real time immediately after surgery or even after a certain period of time, and do not have to visit the hospital with vague fears, leading to a better quality of life. The present disclosure can also help to more quickly and accurately recognize the need to visit the hospital.

Although the present disclosure has been described above using several embodiments, the embodiments are illustrative and not limiting. As such, those of ordinary skill in the technical field to which the present disclosure pertains will understand that various changes and modifications can be made . The scope of the present invention is set forth in the appended claims.

### Industrial Applicability

The present disclosure provides an apparatus for sensing an implant abnormality to minimize a false positive rate and a false negative rate by effectively sensing the abnormality of an implant after a procedure, so the apparatus for sensing an implant abnormality can be applied to medical treatment and plastic surgery using implants.

## Claims

1. Apparatus (100) for sensing an implant abnormality, the apparatus (100) comprising:
a wireless power transmitting unit (110) for wirelessly transmitting power to at least one sensor module (210) mounted on an implant inserted into the human body;
a sensor signal receiving unit (120) for receiving a pressure signal indicating a pressure applied to the implant (200) from said at least one sensor module (210);
a storing unit (130) for storing a predetermined reference pressure value and a first tolerance;
an abnormality determining unit (140) for determining whether the implant (200) is abnormal based on the pressure signal received by the sensor signal receiving unit (120);
a determination signal output unit (150) for outputting the determination signal indicating whether the implant (200) is abnormal;
a control unit (160) for controlling the wireless power transmitting unit (110), the sensor signal receiving unit (120), the storing unit (130), the abnormality determining unit (140), and the determination signal output unit (150); and
a power source unit (170) for supplying the power to the wireless power transmitting unit (110), the sensor signal receiving unit (120), the storing unit (130), the abnormality determining unit (140), and the determination signal output unit (150) through the control unit (160),
wherein the control unit (160) is configured to receive a first pressure signal from said at least one sensor module (210) according to a predetermined period or according to an arbitrary manipulation of a user through the sensor signal receiving unit (120), the apparatus (100) being **characterized**
**in that** abnormality of the implant (200) is determined based on the pressure signal, and the reference pressure value and the first tolerance by the abnormality determining unit (140), and
**in that** the control unit (160) is configured to
compare a first pressure value indicated by the first pressure signal, and a reference pressure value through the abnormality determining unit (140),
receive a second pressure signal from said at least one sensor module (210) after a predetermined first time lapse through the sensor signal receiving unit (120) when an absolute value of a difference between the first pressure value and the reference pressure value is larger than the first tolerance,
compare a second pressure value indicated by the second pressure signal and a reference pressure value through the abnormality determining unit (140), and
output a determination signal indicating that the implant (200) is abnormal through the determination signal output unit (150) when an absolute value of a difference between the second pressure value and the reference pressure value is larger than the first tolerance.

2. Apparatus according to claim 1, wherein the storing unit (130) further stores a second tolerance, and
the control unit is configured to
compare an absolute value of a difference between the first pressure value and the second pressure value, and the second tolerance through the abnormality determining unit (140) when the second pressure value is larger than the reference pressure value, and
output the determination signal indicating whether the implant (200) is abnormal through the determination signal output unit (150) when the absolute value of the difference between the first frst pressure value and the second pressure value is smaller than the second tolerance.

3. Apparatus according to claim 1, wherein said at least one sensor module (210) includes a plurality of sensor modules, and
the storing unit (130) sets and stores the first tolerance differently for each of the plurality of sensor modules.

4. Apparatus according to claim 1, wherein said at least one sensor module (210) includes a plurality of sensor modules, and
the storing unit (130) sets and stores the first tolerance for any one sensor module (210) among the plurality of sensor modules differently from the other sensor modules.

5. Apparatus for sensing an implant abnormality, the apparatus comprising:
a wireless power transmitting unit (110) for wirelessly transmitting power to at least one sensor module (210) mounted on an implant inserted into the human body;
a sensor signal receiving unit (120) for receiving a pressure signal indicating a pressure applied to the implant (200) from said at least one sensor module (210);
a storing unit (130) for storing a predetermined first tolerance;
an abnormality determining unit (140) for determining whether the implant (200) is abnormal based on the pressure signal received by the sensor signal receiving unit (120);
a determination signal output unit (150) for outputting the determination signal indicating whether the implant (200) is abnormal;
a control unit (160) for controlling the wireless power transmitting unit (110), the sensor signal receiving unit (120), the storing unit (130), the abnormality determination unit (140), and the determination signal output unit (150); and
a power source unit (170) for supplying the power to the wireless power transmitting unit (110), the sensor signal receiving unit (120), the storing unit (130), the abnormality determination unit, and the determination signal output unit (150) through the control unit,
wherein the control unit (160) is configured to receive a first pressure signal from said at least one sensor module (210) according to a predetermined period or according to an arbitrary manipulation of a user through the sensor signal receiving unit (120), the apparatus (100) being **characterized**
**in that** abnormality of the implant (200) is determined based on the pressure signal, and the first tolerance by the abnormality determining unit (140), and
**in that** the control unit (160) is configured to
compare a first change amount indicating a difference between a previous value of the first pressure signal and a current value of the first pressure signal, and the first tolerance through the abnormality determining unit (140), and
receive a second pressure signal from said at least one sensor module (210) after a predetermined first time lapse through the sensor signal receiving unit (120) when the first change amount is larger than the first tolerance,
compare a second change amount indicating a difference between a value of the first pressure signal and a value of the second pressure signal, and the first tolerance through the abnormality determining unit (140), and
output the determination signal indicating that the implant (200) is abnormal through the determination signal output unit (150) when the second change amount is larger than the first tolerance.

6. Apparatus according to claim 5, wherein the storing unit (130) further stores a predetermined second tolerance, and
the control unit is configured to
compare an absolute value of a difference between the first change amount and the second change amount, and the second tolerance through the abnormality determining unit (140) when the second change amount is larger than the first tolerance, and
output the determination signal indicating that the implant (200) is abnormal through the determination signal output unit (150) when the absolute value of the difference between the first change amount and the second change amount is smaller than the second tolerance.

7. Apparatus according to claim 5, wherein said at least one sensor module (210) includes a plurality of sensor modules, and
the storing unit (130) sets and stores the first tolerance differently for each of the plurality of sensor modules.

8. Apparatus according to claim 5, wherein said at least one sensor module (210) includes a plurality of sensor modules, and
the storing unit (130) sets and stores the first tolerance for any one sensor module (210) among the plurality of sensor modules differently from the other sensor modules.

9. Apparatus according to any one of claims 1 to 8, wherein the sensor signal receiving unit (120) further receives a biometric signal representing at least one of heart rate, pulse, body temperature, blood pressure, respiratory rate, and blood sugar from said at least one sensor module(210), and
the storing unit (130) further stores the biometric signal received by the sensor signal receiving unit (120).

10. System for sensing an implant abnormality, the system comprising:
a user terminal (500) receiving a pressure signal indicating a pressure applied to an implant from at least one sensor module (210) mounted on the implant (200) inserted into the human body, and transmitting the received pressure signal to the outside; and
a remote server (600) receiving the pressure signal from the user terminal (500), and determining whether the implant (200) is abnormal based on the received pressure signal,
wherein the user terminal (500) includes
a wireless power transmitting unit (510) for wirelessly transmitting power to said at least one sensor module (210),
a sensor signal receiving unit (520) for receiving a pressure signal indicating a pressure applied to the implant (200) from said at least one sensor module (210),
a signal transmitting unit (530) for transmitting the pressure signal received by the sensor signal receiving unit (520) to the remote server (600),
a terminal control unit (540) for controlling the wireless power transmitting unit (510), the sensor signal receiving unit (520), and the signal transmitting unit (530), and
a terminal power source unit (550) for supplying power to the wireless power transmitting unit (510), the sensor signal receiving unit (520), and the signal transmitting unit (530) through the terminal control unit (540),
the remote server (600) includes
a signal receiving unit (610) for receiving a pressure signal from the user terminal (500),
a storing unit (620) for storing a predetermined reference pressure value and a first tolerance,
an abnormality determining unit (630) for determining whether the implant (200) is abnormal based on the pressure signal received from the user terminal (500),
a determination signal output unit (640) for outputting a determination signal indicating whether the implant (200) is abnormal,
a server control unit (650) for controlling the signal receiving unit (610), the storing unit (620), the abnormality determination unit (630), and the determination signal output unit (640), and
a server power source unit (660) for supplying power to the signal receiving unit (610), the storing unit (620), the abnormality determination unit (630), and the determination signal output unit (640) through the server control unit (650), and
the server control unit (650) is configured to receive a first pressure signal from said at least one sensor module (210) according to a predetermined period or according to an arbitrary manipulation of a user through the terminal control unit (540), the system being **characterized**
**in that** abnormality of the implant (200) is determined based on the pressure signal, and the reference pressure value and the first tolerance by the abnormality determining unit, and
**in that** the server control unit (650) is configured to
compare a first pressure value indicated by the first pressure signal, and a reference pressure value through the abnormality determining unit (630),
receive a second pressure signal from said at least one sensor module (210) after a predetermined first time lapse through the terminal control unit (540) when an absolute value of a difference between the first pressure value and the reference pressure value is larger than the first tolerance,
compare a second pressure value indicated by the second pressure signal and a reference pressure value through the abnormality determining unit (630), and
output a determination signal indicating that the implant (200) is abnormal through the determination signal output unit (640) when an absolute value of a difference between the second pressure value and the reference pressure value is larger than the first tolerance.

11. System for sensing an implant abnormality, the system comprising:
a user terminal (500) receiving a pressure signal indicating a pressure applied to an implant from at least one sensor module (210) mounted on the implant (200) inserted into the human body, and transmitting the received pressure signal to the outside; and
a remote server (600) receiving the pressure signal from the user terminal (500), and determining whether the implant (200) is abnormal based on the received pressure signal,
wherein the user terminal (500) includes
a wireless power transmitting unit (510) for wirelessly transmitting power to said at least one sensor module (210),
a sensor signal receiving unit (520) for receiving a pressure signal indicating a pressure applied to the implant (200) from said at least one sensor module (210),
a signal transmitting unit (530) for transmitting the pressure signal received by the sensor signal receiving unit (520) to the remote server (600),
a terminal control unit (540) for controlling the wireless power transmitting unit (510), the sensor signal receiving unit (520), and the signal transmitting unit (530), and
a terminal power source unit (550) for supplying power to the wireless power transmitting unit (510), the sensor signal receiving unit (520), and the signal transmitting unit (530) through the terminal control unit (540),
the remote server (600) includes
a signal receiving unit (610) for receiving a pressure signal from the user terminal (500),
a storing unit (620) for storing a predetermined first tolerance,
an abnormality determining unit (630) for determining whether the implant (200) is abnormal based on the pressure signal received from the user terminal (500),
a determination signal output unit (640) for outputting the determination signal indicating whether the implant (200) is abnormal,
a server control unit (650) for controlling the signal receiving unit (610), the storing unit (620), the abnormality determination unit (630), and the determination signal output unit (640), and
a server power source unit (660) for supplying power to the signal receiving unit (610), the storing unit (620), the abnormality determination unit (630), and the determination signal output unit (640) through the server control unit (650), and
the server control unit (650) is configured to receive a first pressure signal from said at least one sensor module (210) according to a predetermined period or according to an arbitrary manipulation of a user through the terminal control unit (540), the system being **characterized**
**in that** abnormality of the implant (200) is determined based on the pressure signal, and the first tolerance by the abnormality determining unit (630), and
**in that** the server control unit (650) is configured to
compare a first change amount indicating a difference between a previous value of the first pressure signal and a current value of the first pressure signal, and the first tolerance through the abnormality determining unit (630), and
receive a second pressure signal from said at least one sensor module (210) after a predetermined first time lapse through the terminal control unit (540) when the first change amount is larger than the first tolerance,
compares a second change amount indicating a difference between a value of the first pressure signal and a value of the second pressure signal, and the first tolerance through the abnormality determining unit (630), and
output the determination signal indicating that the implant (200) is abnormal through the determination signal output unit (640) when the second change amount is larger than the first tolerance.

12. System according to claim 10 or claim 11, wherein the sensor signal receiving unit (520) further receives a biometric signal representing at least one of heart rate, pulse, body temperature, blood pressure, respiratory rate, and blood sugar from said at least one sensor module (210),
the signal transmitting unit (530) transmits the biometric signal received by the sensor signal receiving unit (520) to the remote server (600),
the signal receiving unit (610) receives the biometric signal from the user terminal (500), and
the storing unit (620) further stores the biometric signal received from the user terminal (500).

## Patentansprüche

1. Einrichtung (100) zum Erfassen einer Implantatanomalie, die Einrichtung (100) umfassend:
eine drahtlose Energieübertragungseinheit (110) zum drahtlosen Übertragen von Energie an mindestens ein Sensormodul (210), das auf einem Implantat angebracht ist, das in den menschlichen Körper eingeführt wird;
eine Sensorsignalempfangseinheit (120) zum Empfangen eines Drucksignals, das einen Druck angibt, der auf das Implantat (200) ausgeübt wird, von dem mindestens einen Sensormodul (210);
eine Speichereinheit (130) zum Speichern eines vorbestimmten Referenzdruckwerts und einer ersten Toleranz;
eine Anomaliebestimmungseinheit (140) zum Bestimmen, ob das Implantat (200) anormal ist, basierend auf dem Drucksignal, das durch die Sensorsignalempfangseinheit (120) empfangen wird;
eine Bestimmungssignalausgabeeinheit (150) zum Ausgeben des Bestimmungssignals, das angibt, ob das Implantat (200) anormal ist;
eine Steuereinheit (160) zum Steuern der drahtlosen Energieübertragungseinheit (110), der Sensorsignalempfangseinheit (120), der Speichereinheit (130), der Anomaliebestimmungseinheit (140) und der Bestimmungssignalausgabeeinheit (150); und
eine Energiequelleneinheit (170) zum Zuführen der Energie zu der drahtlosen Energieübertragungseinheit (110), der Sensorsignalempfangseinheit (120), der Speichereinheit (130), der Anomaliebestimmungseinheit (140) und der Bestimmungssignalausgabeeinheit (150) über die Steuereinheit (160),
wobei die Steuereinheit (160) konfiguriert ist, um ein erstes Drucksignal von dem mindestens einen Sensormodul (210) gemäß einer vorbestimmten Periode oder gemäß einer beliebigen Manipulation eines Benutzers über die Sensorsignalempfangseinheit (120) zu empfangen,
wobei die Einrichtung (100) **dadurch gekennzeichnet ist,**
**dass** eine Anomalie des Implantats (200) durch die Anomaliebestimmungseinheit (140) basierend auf dem Drucksignal und dem Referenzdruckwert und der ersten Toleranz bestimmt wird, und
**dass** die Steuereinheit (160) konfiguriert ist zum Vergleichen eines ersten Druckwerts, der durch das erste Drucksignal angegeben wird, und eines Referenzdruckwerts über die Anomaliebestimmungseinheit (140),
Empfangen eines zweiten Drucksignals von dem mindestens einen Sensormodul (210) nach einem vorbestimmten ersten Zeitablauf über die Sensorsignalempfangseinheit (120), wenn ein Absolutwert eines Unterschieds zwischen dem ersten Druckwert und dem Referenzdruckwert größer als die erste Toleranz ist,
Vergleichen eines zweiten Druckwerts, der durch das zweite Drucksignal angegeben wird, und eines Referenzdruckwerts über die Anomaliebestimmungseinheit (140), und
Ausgeben eines Bestimmungssignals, das angibt, dass das Implantat (200) anormal ist, über die Bestimmungssignalausgabeeinheit (150), wenn ein Absolutwert eines Unterschieds zwischen dem zweiten Druckwert und dem Referenzdruckwert größer als die erste Toleranz ist.

2. Einrichtung nach Anspruch 1, wobei die Speichereinheit (130) ferner eine zweite Toleranz speichert, und
die Steuereinheit konfiguriert ist zum Vergleichen eines Absolutwerts eines Unterschieds zwischen dem ersten Druckwert und dem zweiten Druckwert und der zweiten Toleranz über die Anomaliebestimmungseinheit (140), wenn der zweite Druckwert größer als der Referenzdruckwert ist, und
Ausgeben des Bestimmungssignals, das angibt, ob das Implantat (200) anormal ist, über die Bestimmungssignalausgabeeinheit (150), wenn der Absolutwert des Unterschieds zwischen dem ersten Druckwert und dem zweiten Druckwert kleiner als die zweite Toleranz ist.

3. Einrichtung nach Anspruch 1, wobei das mindestens eine Sensormodul (210) eine Vielzahl von Sensormodulen einschließt, und
die Speichereinheit (130) die erste Toleranz für jedes der Vielzahl von Sensormodulen unterschiedlich festlegt und speichert.

4. Einrichtung nach Anspruch 1, wobei das mindestens eine Sensormodul (210) eine Vielzahl von Sensormodulen einschließt, und
die Speichereinheit (130) die erste Toleranz für ein beliebiges Sensormodul (210) aus der Vielzahl von Sensormodulen unterschiedlich von den anderen Sensormodulen festlegt und speichert.

5. Einrichtung zum Erfassen einer Implantatanomalie, die Einrichtung umfassend:
eine drahtlose Energieübertragungseinheit (110) zum drahtlosen Übertragen von Energie an mindestens ein Sensormodul (210), das auf einem Implantat angebracht ist, das in den menschlichen Körper eingeführt wird;
eine Sensorsignalempfangseinheit (120) zum Empfangen eines Drucksignals, das einen Druck angibt, der auf das Implantat (200) ausgeübt wird, von dem mindestens einen Sensormodul (210);
eine Speichereinheit (130) zum Speichern einer vorbestimmten ersten Toleranz;
eine Anomaliebestimmungseinheit (140) zum Bestimmen, ob das Implantat (200) anormal ist, basierend auf dem Drucksignal, das durch die Sensorsignalempfangseinheit (120) empfangen wird;
eine Bestimmungssignalausgabeeinheit (150) zum Ausgeben des Bestimmungssignals, das angibt, ob das Implantat (200) anormal ist;
eine Steuereinheit (160) zum Steuern der drahtlosen Energieübertragungseinheit (110), der Sensorsignalempfangseinheit (120), der Speichereinheit (130), der Anomaliebestimmungseinheit (140) und der Bestimmungssignalausgabeeinheit (150); und
eine Energiequelleneinheit (170) zum Zuführen der Energie zu der drahtlosen Energieübertragungseinheit (110), der Sensorsignalempfangseinheit (120), der Speichereinheit (130), der Anomaliebestimmungseinheit und der Bestimmungssignalausgabeeinheit (150) über die Steuereinheit,
wobei die Steuereinheit (160) konfiguriert ist, um ein erstes Drucksignal von dem mindestens einen Sensormodul (210) gemäß einer vorbestimmten Periode oder gemäß einer beliebigen Manipulation eines Benutzers über die Sensorsignalempfangseinheit (120) zu empfangen,
wobei die Einrichtung (100) **dadurch gekennzeichnet ist,**
**dass** die Anomalie des Implantats (200) basierend auf dem Drucksignal und der ersten Toleranz durch die Anomaliebestimmungseinheit (140) bestimmt wird, und
**dass** die Steuereinheit (160) konfiguriert ist zum Vergleichen eines ersten Änderungsbetrags, der einen Unterschied zwischen einem vorherigen Wert des ersten Drucksignals und einem aktuellen Wert des ersten Drucksignals angibt, und der ersten Toleranz über die Anomaliebestimmungseinheit (140), und
Empfangen eines zweiten Drucksignals von dem mindestens einen Sensormodul (210) nach einer vorbestimmten ersten Zeitspanne über die Sensorsignalempfangseinheit (120), wenn der erste Änderungsbetrag größer als die erste Toleranz ist,
Vergleichen eines zweiten Änderungsbetrags, der einen Unterschied zwischen einem Wert des ersten Drucksignals und einem Wert des zweiten Drucksignals angibt, und der ersten Toleranz über die Anomaliebestimmungseinheit (140), und
Ausgeben des Bestimmungssignals, das angibt, dass das Implantat (200) anormal ist, über die Bestimmungssignalausgabeeinheit (150), wenn der zweite Änderungsbetrag größer als die erste Toleranz ist.

6. Einrichtung nach Anspruch 5, wobei die Speichereinheit (130) ferner eine vorbestimmte zweite Toleranz speichert, und
die Steuereinheit konfiguriert ist zum Verleichen eines Absolutwerts eines Unterschieds zwischen dem ersten Änderungsbetrag und dem zweiten Änderungsbetrag und der zweiten Toleranz über die Anomaliebestimmungseinheit (140), wenn der zweite Änderungsbetrag größer als die erste Toleranz ist, und
Ausgeben des Bestimmungssignals, das angibt, dass das Implantat (200) anormal ist, über die Bestimmungssignalausgabeeinheit (150), wenn der absolute Wert des Unterschieds zwischen dem ersten Änderungsbetrag und dem zweiten Änderungsbetrag kleiner als die zweite Toleranz ist.

7. Einrichtung nach Anspruch 5, wobei das mindestens eine Sensormodul (210) eine Vielzahl von Sensormodulen einschließt, und
die Speichereinheit (130) die erste Toleranz für jedes der Vielzahl von Sensormodulen unterschiedlich festlegt und speichert.

8. Einrichtung nach Anspruch 5, wobei das mindestens eine Sensormodul (210) eine Vielzahl von Sensormodulen einschließt, und
die Speichereinheit (130) die erste Toleranz für ein beliebiges Sensormodul (210) aus der Vielzahl von Sensormodulen unterschiedlich von den anderen Sensormodulen festlegt und speichert.

9. Einrichtung nach einem der Ansprüche 1 bis 8, wobei die Sensorsignalempfangseinheit (120) ferner ein biometrisches Signal, das mindestens eines von Herzfrequenz, Puls, Körpertemperatur, Blutdruck, Atemfrequenz und Blutzucker darstellt, von dem mindestens einen Sensormodul (210) empfängt, und
die Speichereinheit (130) ferner das biometrische Signal speichert, das durch die Sensorsignalempfangseinheit (120) empfangen wird.

10. System zum Erfassen einer Implantatanomalie, das System umfassend:
ein Benutzerendgerät (500), das ein Drucksignal, das einen Druck angibt, der auf ein Implantat ausgeübt wird, von mindestens einem Sensormodul (210) empfängt, das auf dem Implantat (200) angebracht ist, das in den menschlichen Körper eingeführt wird, und das empfangene Drucksignal nach außen überträgt; und
einen entfernten Server (600), der das Drucksignal von dem Benutzerendgerät (500) empfängt und basierend auf dem empfangenen Drucksignal bestimmt, ob das Implantat (200) anormal ist,
wobei das Benutzerendgerät (500) einschließt
eine drahtlose Energieübertragungseinheit (510) zum drahtlosen Übertragen von Energie an das mindestens eine Sensormodul (210),
eine Sensorsignalempfangseinheit (520) zum Empfangen eines Drucksignals, das einen Druck angibt, der auf das Implantat (200) ausgeübt wird, von dem mindestens einen Sensormodul (210),
eine Signalübertragungseinheit (530) zum Übertragen des Drucksignals, das durch die Sensorsignalempfangseinheit (520) empfangen wird, an den entfernten Server (600),
eine Endgerätesteuereinheit (540) zum Steuern der drahtlosen Energieübertragungseinheit (510), der Sensorsignalempfangseinheit (520) und der Signalübertragungseinheit (530), und
eine Endgeräteenergiequelleneinheit (550) zum Zuführen von Energie zu der drahtlosen Energieübertragungseinheit (510), der Sensorsignalempfangseinheit (520) und der Signalübertragungseinheit (530) über die Endgerätesteuereinheit (540),
der entfernte Server (600) einschließt
eine Signalempfangseinheit (610) zum Empfangen eines Drucksignals von dem Benutzerendgerät (500),
eine Speichereinheit (620) zum Speichern eines vorbestimmten Referenzdruckwerts und einer ersten Toleranz,
eine Anomaliebestimmungseinheit (630) zum Bestimmen, ob das Implantat (200) anomal ist, basierend auf dem Drucksignal, das von dem Benutzerendgerät (500) empfangen wird,
eine Bestimmungssignalausgabeeinheit (640) zum Ausgeben eines Bestimmungssignals, das angibt, ob das Implantat (200) anormal ist,
eine Serversteuereinheit (650) zum Steuern der Signalempfangseinheit (610), der Speichereinheit (620), der Anomaliebestimmungseinheit (630) und der Bestimmungssignalausgabeeinheit (640), und
eine Serverenergiequelleneinheit (660) zum Zuführen von Energie zu der Signalempfangseinheit (610), der Speichereinheit (620), der Anomaliebestimmungseinheit (630) und der Bestimmungssignalausgabeeinheit (640) über die Serversteuereinheit (650), und
die Serversteuereinheit (650) konfiguriert ist, um ein erstes Drucksignal von dem mindestens einen Sensormodul (210) gemäß einer vorbestimmten Periode oder gemäß einer beliebigen Manipulation eines Benutzers über die Endgerätesteuereinheit (540) zu empfangen,
wobei das System **dadurch gekennzeichnet ist,**
**dass** die Anomalie des Implantats (200) basierend auf dem Drucksignal und dem Referenzdruckwert und der ersten Toleranz durch die Anomaliebestimmungseinheit bestimmt wird, und
**dass** die Serversteuereinheit (650) konfiguriert ist zum Vergleichen eines ersten Druckwerts, der durch das erste Drucksignal angegeben wird, und eines Referenzdruckwerts über die Anomaliebestimmungseinheit (630),
Empfangen eines zweiten Drucksignals von dem mindestens einen Sensormodul (210) nach einer vorbestimmten ersten Zeitspanne über die Endgerätesteuereinheit (540), wenn ein absoluter Wert eines Unterschieds zwischen dem ersten Druckwert und dem Referenzdruckwert größer als die erste Toleranz ist,
Vergleichen eines zweiten Druckwerts, der durch das zweite Drucksignal angegeben wird, und eines Referenzdruckwerts über die Anomaliebestimmungseinheit (630), und
Ausgeben eines Bestimmungssignals, das angibt, dass das Implantat (200) anormal ist, über die Bestimmungssignalausgabeeinheit (640), wenn ein Absolutwert eines Unterschieds zwischen dem zweiten Druckwert und dem Referenzdruckwert größer als die erste Toleranz ist.

11. System zum Erfassen einer Implantatanomalie, das System umfassend:
ein Benutzerendgerät (500), das ein Drucksignal, das einen Druck angibt, der auf ein Implantat ausgeübt wird, von mindestens einem Sensormodul (210) empfängt, das auf dem Implantat (200) angebracht ist, das in den menschlichen Körper eingeführt wird, und das empfangene Drucksignal nach außen überträgt; und
einen entfernten Server (600), der das Drucksignal von dem Benutzerendgerät (500) empfängt und basierend auf dem empfangenen Drucksignal bestimmt, ob das Implantat (200) anormal ist,
wobei das Benutzerendgerät (500) einschließt
eine drahtlose Energieübertragungseinheit (510) zum drahtlosen Übertragen von Energie an das mindestens eine Sensormodul (210),
eine Sensorsignalempfangseinheit (520) zum Empfangen eines Drucksignals, das einen Druck angibt, der auf das Implantat (200) ausgeübt wird, von dem mindestens einen Sensormodul (210),
eine Signalübertragungseinheit (530) zum Übertragen des Drucksignals, das durch die Sensorsignalempfangseinheit (520) empfangen wird, an den entfernten Server (600),
eine Endgerätesteuereinheit (540) zum Steuern der drahtlosen Energieübertragungseinheit (510), der Sensorsignalempfangseinheit (520) und der Signalübertragungseinheit (530), und
eine Endgeräteenergiequelleneinheit (550) zum Zuführen von Energie zu der drahtlosen Energieübertragungseinheit (510), der Sensorsignalempfangseinheit (520) und der Signalübertragungseinheit (530) über die Endgerätesteuereinheit (540),
der entfernte Server (600) einschließt
eine Signalempfangseinheit (610) zum Empfangen eines Drucksignals von dem Benutzerendgerät (500),
eine Speichereinheit (620) zum Speichern einer vorbestimmten ersten Toleranz,
eine Anomaliebestimmungseinheit (630) zum Bestimmen, ob das Implantat (200) anomal ist, basierend auf dem Drucksignal, das von dem Benutzerendgerät (500) empfangen wird,
eine Bestimmungssignalausgabeeinheit (640) zum Ausgeben des Bestimmungssignals, das angibt, ob das Implantat (200) anormal ist,
eine Serversteuereinheit (650) zum Steuern der Signalempfangseinheit (610), der Speichereinheit (620), der Anomaliebestimmungseinheit (630) und der Bestimmungssignalausgabeeinheit (640), und
eine Serverenergiequelleneinheit (660) zum Zuführen von Energie zu der Signalempfangseinheit (610), der Speichereinheit (620), der Anomaliebestimmungseinheit (630) und der Bestimmungssignalausgabeeinheit (640) über die Serversteuereinheit (650), und
die Serversteuereinheit (650) konfiguriert ist, um ein erstes Drucksignal von dem mindestens einen Sensormodul (210) gemäß einer vorbestimmten Periode oder gemäß einer beliebigen Manipulation eines Benutzers über die Endgerätesteuereinheit (540) zu empfangen,
wobei das System **dadurch gekennzeichnet ist**,
**dadurch, dass** die Anomalie des Implantats (200) basierend auf dem Drucksignal und der ersten Toleranz durch die Anomaliebestimmungseinheit (630) bestimmt wird, und
**dass** die Serversteuereinheit (650) konfiguriert ist zum Vergleichen eines ersten Änderungsbetrags, der einen Unterschied zwischen einem vorherigen Wert des ersten Drucksignals und einem aktuellen Wert des ersten Drucksignals angibt, und der ersten Toleranz über die Anomaliebestimmungseinheit (630), und
Empfangen eines zweiten Drucksignals von dem mindestens einen Sensormodul (210) nach einem vorbestimmten ersten Zeitablauf über die Endgerätesteuereinheit (540), wenn der erste Änderungsbetrag größer als die erste Toleranz ist,
Vergleichen eines zweiten Änderungsbetrags, der einen Unterschied zwischen einem Wert des ersten Drucksignals und einem Wert des zweiten Drucksignals angibt, und der ersten Toleranz über die Anomaliebestimmungseinheit (630), und
Ausgeben des Bestimmungssignals, das angibt, dass das Implantat (200) anormal ist, über die Bestimmungssignalausgabeeinheit (640), wenn der zweite Änderungsbetrag größer als die erste Toleranz ist.

12. System nach Anspruch 10 oder 11, wobei die Sensorsignalempfangseinheit (520) ferner ein biometrisches Signal, das mindestens eines von Herzfrequenz, Puls, Körpertemperatur, Blutdruck, Atemfrequenz und Blutzucker darstellt, von dem mindestens einen Sensormodul (210) empfängt,
die Signalübertragungseinheit (530) das biometrische Signal, das durch die Sensorsignalempfangseinheit (520) empfangen wird, an den entfernten Server (600) überträgt,
die Signalempfangseinheit (610) das biometrische Signal von dem Benutzerendgerät (500) empfängt, und
die Speichereinheit (620) ferner das biometrische Signal speichert, das von dem Benutzerendgerät (500) empfangen wird.

## Revendications

1. Appareil (100) permettant de détecter une anomalie d'implant, l'appareil (100) comprenant :
une unité de transmission d'énergie sans fil (110) pour transmettre de l'énergie sans fil à au moins un module de capteur (210) monté sur un implant inséré dans le corps humain ;
une unité de réception de signal de capteur (120) pour recevoir un signal de pression indiquant une pression appliquée à l'implant (200) en provenance dudit au moins un module de capteur (210) ;
une unité de stockage (130) pour stocker une valeur de pression de référence prédéterminée et une première tolérance ;
une unité de détermination d'anomalie (140) pour déterminer si l'implant (200) est anormal en fonction du signal de pression reçu par l'unité de réception de signal de capteur (120) ;
une unité de sortie de signal de détermination (150) pour délivrer en sortie le signal de détermination indiquant si l'implant (200) est anormal ;
une unité de commande (160) pour commander l'unité de transmission d'énergie sans fil (110), l'unité de réception de signal de capteur (120), l'unité de stockage (130), l'unité de détermination d'anomalie (140), et l'unité de sortie de signal de détermination (150) ; et
une unité de source d'énergie (170) pour fournir l'énergie à l'unité de transmission d'énergie sans fil (110), à l'unité de réception de signal de capteur (120), à l'unité de stockage (130), à l'unité de détermination d'anomalie (140), et à l'unité de sortie de signal de détermination (150) à travers l'unité de commande (160),
dans lequel l'unité de commande (160) est configurée pour recevoir un premier signal de pression en provenance dudit au moins un module de capteur (210) selon une période prédéterminée ou selon une manipulation arbitraire d'un utilisateur à travers l'unité de réception de signal de capteur (120),
l'appareil (100) étant **caractérisé**
**en ce qu'**une anomalie de l'implant (200) est déterminée en fonction du signal de pression, et de la valeur de pression de référence et de la première tolérance par l'unité de détermination d'anomalie (140), et
**en ce que** l'unité de commande (160) est configurée pour comparer une première valeur de pression indiquée par le premier signal de pression, et une valeur de pression de référence à travers l'unité de détermination d'anomalie (140),
recevoir un second signal de pression en provenance dudit au moins un module de capteur (210) après un premier laps de temps prédéterminé à travers l'unité de réception de signal de capteur (120) lorsqu'une valeur absolue d'une différence entre la première valeur de pression et la valeur de pression de référence est supérieure à la première tolérance,
comparer une seconde valeur de pression indiquée par le second signal de pression et une valeur de pression de référence à travers l'unité de détermination d'anomalie (140), et
délivrer en sortie un signal de détermination indiquant que l'implant (200) est anormal à travers l'unité de sortie de signal de détermination (150) lorsqu'une valeur absolue d'une différence entre la seconde valeur de pression et la valeur de pression de référence est supérieure à la première tolérance.

2. Appareil selon la revendication 1, dans lequel l'unité de stockage (130) stocke en outre une seconde tolérance, et
l'unité de commande est configurée pour comparer une valeur absolue d'une différence entre la première valeur de pression et la seconde valeur de pression, et la seconde tolérance à travers l'unité de détermination d'anomalie (140) lorsque la seconde valeur de pression est supérieure à la valeur de pression de référence, et
délivrer en sortie le signal de détermination indiquant si l'implant (200) est anormal à travers l'unité de sortie de signal de détermination (150) lorsque la valeur absolue de la différence entre la première valeur de pression et la seconde valeur de pression est inférieure à la seconde tolérance.

3. Appareil selon la revendication 1, dans lequel ledit au moins un module de capteur (210) comporte une pluralité de modules de capteur, et
l'unité de stockage (130) définit et stocke la première tolérance différemment pour chacun de la pluralité de modules de capteur.

4. Appareil selon la revendication 1, dans lequel ledit au moins un module de capteur (210) comporte une pluralité de modules de capteur, et
l'unité de stockage (130) définit et stocke la première tolérance pour un quelconque module de capteur (210) parmi la pluralité de modules de capteur différemment des autres modules de capteur.

5. Appareil permettant de détecter une anomalie d'implant, l'appareil comprenant :
une unité de transmission d'énergie sans fil (110) pour transmettre de l'énergie sans fil à au moins un module de capteur (210) monté sur un implant inséré dans le corps humain ;
une unité de réception de signal de capteur (120) pour recevoir un signal de pression indiquant une pression appliquée à l'implant (200) en provenance dudit au moins un module de capteur (210) ;
une unité de stockage (130) pour stocker une première tolérance prédéterminée ;
une unité de détermination d'anomalie (140) pour déterminer si l'implant (200) est anormal en fonction du signal de pression reçu par l'unité de réception de signal de capteur (120) ;
une unité de sortie de signal de détermination (150) pour délivrer en sortie le signal de détermination indiquant si l'implant (200) est anormal ;
une unité de commande (160) pour commander l'unité de transmission d'énergie sans fil (110), l'unité de réception de signal de capteur (120), l'unité de stockage (130), l'unité de détermination d'anomalie (140), et l'unité de sortie de signal de détermination (150) ; et
une unité de source d'énergie (170) pour fournir l'énergie à l'unité de transmission d'énergie sans fil (110), à l'unité de réception de signal de capteur (120), à l'unité de stockage (130), à l'unité de détermination d'anomalie, et à l'unité de sortie de signal de détermination (150) à travers l'unité de commande,
dans lequel l'unité de commande (160) est configurée pour recevoir un premier signal de pression en provenance dudit au moins un module de capteur (210) selon une période prédéterminée ou selon une manipulation arbitraire d'un utilisateur à travers l'unité de réception de signal de capteur (120),
l'appareil (100) étant **caractérisé**
**en ce qu'**une anomalie de l'implant (200) est déterminée en fonction du signal de pression, et de la première tolérance par l'unité de détermination d'anomalie (140), et
**en ce que** l'unité de commande (160) est configurée pour comparer une première quantité de variation indiquant une différence entre une valeur précédente du premier signal de pression et une valeur actuelle du premier signal de pression, et la première tolérance à travers l'unité de détermination d'anomalie (140), et
recevoir un second signal de pression en provenance dudit au moins un module de capteur (210) après un premier laps de temps prédéterminé à travers l'unité de réception de signal de capteur (120) lorsque la première quantité de variation est supérieure à la première tolérance,
comparer une seconde quantité de variation indiquant une différence entre une valeur du premier signal de pression et une valeur du second signal de pression, et la première tolérance à travers l'unité de détermination d'anomalie (140), et
délivrer en sortie le signal de détermination indiquant que l'implant (200) est anormal à travers l'unité de sortie de signal de détermination (150) lorsque la seconde quantité de variation est supérieure à la première tolérance.

6. Appareil selon la revendication 5, dans lequel l'unité de stockage (130) stocke en outre une seconde tolérance prédéterminée, et
l'unité de commande est configurée pour comparer une valeur absolue d'une différence entre la première quantité de variation et la seconde quantité de variation, et la seconde tolérance à travers l'unité de détermination d'anomalie (140) lorsque la seconde quantité de variation est supérieure à la première tolérance, et
délivrer en sortie le signal de détermination indiquant que l'implant (200) est anormal à travers l'unité de sortie de signal de détermination (150) lorsque la valeur absolue de la différence entre la première quantité de variation et la seconde quantité de variation est inférieure à la seconde tolérance.

7. Appareil selon la revendication 5, dans lequel ledit au moins un module de capteur (210) comporte une pluralité de modules de capteur, et
l'unité de stockage (130) définit et stocke la première tolérance différemment pour chacun de la pluralité de modules de capteur.

8. Appareil selon la revendication 5, dans lequel ledit au moins un module de capteur (210) comporte une pluralité de modules de capteur, et
l'unité de stockage (130) définit et stocke la première tolérance pour un quelconque module de capteur (210) parmi la pluralité de modules de capteur différemment des autres modules de capteur.

9. Appareil selon l'une quelconque des revendications 1 à 8, dans lequel l'unité de réception de signal de capteur (120) reçoit en outre un signal biométrique représentant au moins l'un parmi une fréquence cardiaque, un pouls, une température corporelle, une pression artérielle, une fréquence respiratoire, et une glycémie en provenance dudit au moins un module de capteur (210), et
l'unité de stockage (130) stocke en outre le signal biométrique reçu par l'unité de réception de signal de capteur (120).

10. Système permettant de détecter une anomalie d'implant, le système comprenant :
un terminal utilisateur (500) recevant un signal de pression indiquant une pression appliquée à un implant en provenance d'au moins un module de capteur (210) monté sur l'implant (200) inséré dans le corps humain, et transmettant le signal de pression reçu vers l'extérieur ; et
un serveur distant (600) recevant le signal de pression en provenance du terminal utilisateur (500), et déterminant si l'implant (200) est anormal en fonction du signal de pression reçu,
dans lequel le terminal utilisateur (500) comporte
une unité de transmission d'énergie sans fil (510) pour transmettre de l'énergie sans fil audit au moins un module de capteur (210),
une unité de réception de signal de capteur (520) pour recevoir un signal de pression indiquant une pression appliquée à l'implant (200) en provenance dudit au moins un module de capteur (210),
une unité de transmission de signal (530) pour transmettre le signal de pression reçu par l'unité de réception de signal de capteur (520) au serveur distant (600),
une unité de commande de terminal (540) pour commander l'unité de transmission d'énergie sans fil (510), l'unité de réception de signal de capteur (520), et l'unité de transmission de signal (530), et
une unité de source d'énergie de terminal (550) pour fournir de l'énergie à l'unité de transmission d'énergie sans fil (510), à l'unité de réception de signal de capteur (520), et à l'unité de transmission de signal (530) à travers l'unité de commande de terminal (540),
le serveur distant (600) comporte
une unité de réception de signal (610) pour recevoir un signal de pression en provenance du terminal utilisateur (500),
une unité de stockage (620) pour stocker une valeur de pression de référence prédéterminée et une première tolérance,
une unité de détermination d'anomalie (630) pour déterminer si l'implant (200) est anormal en fonction du signal de pression reçu en provenance du terminal utilisateur (500),
une unité de sortie de signal de détermination (640) pour délivrer en sortie un signal de détermination indiquant si l'implant (200) est anormal,
une unité de commande de serveur (650) pour commander l'unité de réception de signal (610), l'unité de stockage (620), l'unité de détermination d'anomalie (630), et l'unité de sortie de signal de détermination (640), et
une unité de source d'énergie de serveur (660) pour fournir de l'énergie à l'unité de réception de signal (610), à l'unité de stockage (620), à l'unité de détermination d'anomalie (630), et à l'unité de sortie de signal de détermination (640) à travers l'unité de commande de serveur (650), et
l'unité de commande de serveur (650) est configurée pour recevoir un premier signal de pression en provenance dudit au moins un module de capteur (210) selon une période prédéterminée ou selon une manipulation arbitraire d'un utilisateur à travers l'unité de commande de terminal (540),
le système étant **caractérisé**
**en ce qu'**une anomalie de l'implant (200) est déterminée en fonction du signal de pression, et de la valeur de pression de référence et de la première tolérance par l'unité de détermination d'anomalie, et
**en ce que** l'unité de commande de serveur (650) est configurée pour comparer une première valeur de pression indiquée par le premier signal de pression, et une valeur de pression de référence à travers l'unité de détermination d'anomalie (630),
recevoir un second signal de pression en provenance dudit au moins un module de capteur (210) après un premier laps de temps prédéterminé à travers l'unité de commande de terminal (540) lorsqu'une valeur absolue d'une différence entre la première valeur de pression et la valeur de pression de référence est supérieure à la première tolérance,
comparer une seconde valeur de pression indiquée par le second signal de pression et une valeur de pression de référence à travers l'unité de détermination d'anomalie (630), et
délivrer en sortie un signal de détermination indiquant que l'implant (200) est anormal à travers l'unité de sortie de signal de détermination (640) lorsqu'une valeur absolue d'une différence entre la seconde valeur de pression et la valeur de pression de référence est supérieure à la première tolérance.

11. Système permettant de détecter une anomalie d'implant, le système comprenant :
un terminal utilisateur (500) recevant un signal de pression indiquant une pression appliquée à un implant en provenance d'au moins un module de capteur (210) monté sur l'implant (200) inséré dans le corps humain, et transmettant le signal de pression reçu vers l'extérieur ; et
un serveur distant (600) recevant le signal de pression en provenance du terminal utilisateur (500), et déterminant si l'implant (200) est anormal en fonction du signal de pression reçu,
dans lequel le terminal utilisateur (500) comporte
une unité de transmission d'énergie sans fil (510) pour transmettre de l'énergie sans fil audit au moins un module de capteur (210),
une unité de réception de signal de capteur (520) pour recevoir un signal de pression indiquant une pression appliquée à l'implant (200) en provenance dudit au moins un module de capteur (210),
une unité de transmission de signal (530) pour transmettre le signal de pression reçu par l'unité de réception de signal de capteur (520) au serveur distant (600),
une unité de commande de terminal (540) pour commander l'unité de transmission d'énergie sans fil (510), l'unité de réception de signal de capteur (520), et l'unité de transmission de signal (530), et
une unité de source d'énergie de terminal (550) pour fournir de l'énergie à l'unité de transmission d'énergie sans fil (510), à l'unité de réception de signal de capteur (520), et à l'unité de transmission de signal (530) à travers l'unité de commande de terminal (540),
le serveur distant (600) comporte
une unité de réception de signal (610) pour recevoir un signal de pression en provenance du terminal utilisateur (500),
une unité de stockage (620) pour stocker une première tolérance prédéterminée,
une unité de détermination d'anomalie (630) pour déterminer si l'implant (200) est anormal en fonction du signal de pression reçu en provenance du terminal utilisateur (500),
une unité de sortie de signal de détermination (640) pour délivrer en sortie le signal de détermination indiquant si l'implant (200) est anormal,
une unité de commande de serveur (650) pour commander l'unité de réception de signal (610), l'unité de stockage (620), l'unité de détermination d'anomalie (630), et l'unité de sortie de signal de détermination (640), et
une unité de source d'énergie de serveur (660) pour fournir de l'énergie à l'unité de réception de signal (610), à l'unité de stockage (620), à l'unité de détermination d'anomalie (630), et à l'unité de sortie de signal de détermination (640) à travers l'unité de commande de serveur (650), et
l'unité de commande de serveur (650) est configurée pour recevoir un premier signal de pression en provenance dudit au moins un module de capteur (210) selon une période prédéterminée ou selon une manipulation arbitraire d'un utilisateur à travers l'unité de commande de terminal (540),
le système étant **caractérisé**
**en ce qu'**une anomalie de l'implant (200) est déterminée en fonction du signal de pression, et de la première tolérance par l'unité de détermination d'anomalie (630), et
**en ce que** l'unité de commande de serveur (650) est configurée pour comparer une première quantité de variation indiquant une différence entre une valeur précédente du premier signal de pression et une valeur actuelle du premier signal de pression, et la première tolérance à travers l'unité de détermination d'anomalie (630), et
recevoir un second signal de pression en provenance dudit au moins un module de capteur (210) après un premier laps de temps prédéterminé à travers l'unité de commande de terminal (540) lorsque la première quantité de variation est supérieure à la première tolérance,
comparer une seconde quantité de variation indiquant une différence entre une valeur du premier signal de pression et une valeur du second signal de pression, et la première tolérance à travers l'unité de détermination d'anomalie (630), et
délivrer en sortie le signal de détermination indiquant que l'implant (200) est anormal à travers l'unité de sortie de signal de détermination (640) lorsque la seconde quantité de variation est supérieure à la première tolérance.

12. Système selon la revendication 10 ou la revendication 11, dans lequel l'unité de réception de signal de capteur (520) reçoit en outre un signal biométrique représentant au moins l'un parmi une fréquence cardiaque, un pouls, une température corporelle, une pression artérielle, une fréquence respiratoire, et une glycémie en provenance dudit au moins un module de capteur (210),
l'unité de transmission de signal (530) transmet le signal biométrique reçu par l'unité de réception de signal de capteur (520) au serveur distant (600),
l'unité de réception de signal (610) reçoit le signal biométrique en provenance du terminal utilisateur (500), et
l'unité de stockage (620) stocke en outre le signal biométrique reçu en provenance du terminal utilisateur (500).
